(19) Europäisches Patentamt
European Patent Office
Office européen des brevets

(11) EP 3 088 938 A1

(12) EUROPEAN PATENT APPLICATION
published in accordance with Art. 153(4) EPC

(43) Date of publication:
02.11.2016 Bulletin 2016/44

(51) Int Cl.:
G02C 13/00 (2006.01)     G02C 7/06 (2006.01)

(21) Application number: 14873345.4

(86) International application number:
PCT/JP2014/084550

(22) Date of filing: 26.12.2014

(87) International publication number:
WO 2015/099135 (02.07.2015 Gazette 2015/26)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 26.12.2013   JP 2013269362
31.03.2014   JP 2014070974

(71) Applicant: Hoya Lens Thailand Ltd.
Patumthani (TH)

(72) Inventor: HATANAKA, Takashi
Tokyo 1608347 (JP)

(74) Representative: Betten & Resch
Patent- und Rechtsanwälte PartGmbB
Maximiliansplatz 14
80333 München (DE)

(54) METHOD, PROGRAM, AND DEVICE FOR MANUFACTURING PROGRESSIVE REFRACTIVE POWER LENS, PROGRESSIVE REFRACTIVE POWER LENS MANUFACTURING METHOD, AND LENS SUPPLY SYSTEM

(57) Provided is a method including a blur sensitivity estimating step for estimating a blur sensitivity indicating a sensitivity of a spectacles wearer relative to a blur at the time when the spectacles wearer looks at an object through lenses having a prescription power of a first refractive unit, a first area adjusting step for adjusting an area of the first refractive unit based on the blur sensitivity estimated in the blur sensitivity estimating step, a design reference lens selecting step for selecting at least a single basic design lens for coinciding with or approximating to the lens of which the area of the first refractive unit is adjusted in the first area adjusting step from among a predetermined basic design lens group as a design reference lens of the progressive lens to be worn by the spectacles wearer, and a data creating step for creating data to manufacture the progressive lens based on a lens design of the selected design reference lens.

FIG. 6

**Description**

Technical Field

The present invention relates to a method, a program, and a device for manufacturing a progressive refractive power lens (referred to as "progressive lens" below), a manufacturing method for the progressive lens, and a lens supply system.

Background Art

When spectacles are created at a spectacles store, to improve vision of a wearer at the time when the wearer wears the spectacles, a prescription power of the wearer is measured (for example, JP 2002-200041 A).

For example, an inspector who performs prescription measurement measures a temporary prescription power by using a device for objectively measuring a refraction power of an eye such as an autorefractometer. Subsequently, the inspector selects a trial lens in reference to the measuring result (temporary prescription power) and arranges the trial lens in front of the eye of the expected wearer by using a phoropter or a test frame and makes the expected wearer visually recognize an optotype (visual target) through the arranged trial lens. The inspector asks the expected wearer how the optotype, which has been visually recognized, is looked and replaces the trial lens according to the result. The inspector repeats these works. The optometric work is continued until a value of a power to obtain the highest visual acuity (full corrected value) is found. During the optometric work performed by using the trial lens, a power on the most positive side from among the powers to obtain the highest visual acuity is assumed to be the full corrected value.

When the full corrected value of the expected wearer is found, the inspector makes the expected wearer wear a test frame to which the trial lens having the full corrected value or the value obtained by adjusting the full corrected value as the temporary prescription power is fitted for a certain period of time. Accordingly, the expected wearer determines whether the expected wearer can continuously and comfortably wear the spectacles with the temporary prescription power. After wearing the spectacles with the temporary prescription power for a certain period of time, for example, the expected wearer orders to slightly weaken the power because a burden is applied to the eyes since the vision of the expected wearer is too clear or orders to slightly strengthen the power to obtain the power which makes vision of the expected wearer more clear. In consideration of the order of the expected wearer and experimental judgment, the inspector continues the optometric work. The optometric work is continued until the expected wearer has no more request and the final prescription power is determined.

Summary of Invention

In this way, since the prescription power is determined in consideration of the subjective view of the expected wearer, the prescription power does not necessarily coincide with the full corrected value. Especially, when the type, in which the refractive power changes in the lens, such as the progressive lens is used, the inconsistency between the prescription power and the full corrected value may cause a problem in the vision through the spectacle lens. Specifically, regarding the progressive lens, aberration is allocated over the lens. Therefore, when the prescription power is deviated from the full corrected value, unintended aberration is generated. Also, the vision of the expected wearer through the spectacles is different from that in a case where the spectacles are prescribed with the power close to the full corrected value. Therefore, for example, when the expected wearer wears the spectacle lens created with the prescription power apart from the full corrected value, the expected wearer may feel unsatisfied about the vision.

Here, how the person perceives a blurred image and a sharpened image (blur sensitivity indicating sensitivity relative to blur) is different from person to person. Specifically, the blur sensitivity is different depending on the size of the pupil diameter, transparency of an ocular media in an eyeball, an ability to form a perceived image in the brain. As a result of intensive studies, the inventor obtains knowledge such that after the blur sensitivity caused by the prescription power determined by the optometric work is obtained, the spectacle lens which can reduce the dissatisfaction can be selected and designed based on the obtained blur sensitivity.

A device for manufacturing a progressive lens according to the present embodiment in consideration of the above includes a blur sensitivity estimating unit which estimates a blur sensitivity indicating a sensitivity of a spectacles wearer relative to a blur at the time when the spectacles wearer looks at an object through lenses having a prescription power of a first refractive unit, a first area adjusting unit which adjusts an area of a clear-visible region of the first refractive unit based on the blur sensitivity estimated by the blur sensitivity estimating unit, a design reference lens selecting unit which selects at least a single basic design lens for coinciding with or approximating to the lens of which the area of the clear-visible region of the first refractive unit is adjusted by the first area adjusting unit from among a predetermined basic design lens group as a design reference lens of the progressive lens to be worn by the spectacles wearer, and a data creating unit which creates data to manufacture the progressive lens based on a lens design of the design reference lens selected by the design reference lens selecting unit. The progressive lens includes the first refractive unit having a first refractive power, a second refractive unit having a second refractive power in which a refractive power (addition power) is applied to first refractive power the for near vision, and a corridor in which the refractive power is progressively changed along a meridian from the first refractive unit to the second refractive unit.

According to the present embodiment, since the basic design lens of which the area of the clear-visible region of the first refractive unit is appropriately adjusted according to the blur sensitivity different for each person is selected as the design reference lens of the progressive lens to be worn by the spectacles wearer, the progressive lens which can reduce the dissatisfaction can be designed.

For example, the blur sensitivity is estimated based on a relation between the full corrected value of the spectacles wearer and a value of the prescription power of the first refractive unit.

In the first area adjusting step, the area of the clear-visible region of the first refractive unit may be adjusted based on the blur sensitivity estimated by the blur sensitivity estimating unit or the visual acuity obtained by using the prescription power of the first refractive unit.

The device for manufacturing the progressive lens according to the present embodiment may include a second area adjusting unit for adjusting an area of a clear-visible region of the second refractive unit based on a visual acuity obtained by using the prescription power of the second refractive unit. In this case, the design reference lens selecting unit selects at least a single basic design lens for coinciding with or approximating to the lens of which the area of the clear-visible region of the first refractive unit is adjusted by the first area adjusting unit and for coinciding with or approximating to the lens of which the area of the clear-visible region of the second refractive unit is adjusted by the second area adjusting unit from among a predetermined basic design lens group as the design reference lens of the progressive lens to be worn by the spectacles wearer.

The device for manufacturing the progressive lens according to the present embodiment may include an input unit for requesting inputs of the full corrected value and the value of the prescription power of the first refractive unit. In this case, the blur sensitivity estimating unit estimates the blur sensitivity based on a relation between the full corrected value input via the input unit and the value of the prescription power of the first refractive unit input via the input unit.

The input unit may request an input of the visual acuity obtained by using the prescription power of the first refractive unit. In this case, the first area adjusting unit adjusts the area of the clear-visible region of the first refractive unit based on the blur sensitivity estimated by the blur sensitivity estimating unit and the visual acuity obtained by using the prescription power of the first refractive unit input via the input unit.

A lens supply system for supplying a progressive lens according to the present embodiment includes the device for manufacturing the progressive lens and a biological signal measuring device for measuring a biological signal of the spectacles wearer according to sharpness of a presented image. In this system, the blur sensitivity is estimated based on the biological signal measured by the biological signal measuring device.

Also, the lens supply system for supplying the progressive lens according to the present embodiment includes the device for manufacturing the progressive lens and an input device connected to the device via a predetermined network. The input device includes an input unit for requesting an input of information to design the progressive lens and a transmission unit for transmitting the information input via the input unit to the device. The blur sensitivity estimating unit estimates the blur sensitivity based on the information transmitted by the transmission unit, and the first area adjusting unit adjusts the area of the first refractive unit based on the blur sensitivity estimated by the blur sensitivity estimating unit. The design reference lens selecting unit selects at least a single basic design lens for coinciding with or approximating to the lens of which the area of the first refractive unit is adjusted by the first area adjusting unit from among the predetermined basic design lens group as the design reference lens of the progressive lens to be worn by the spectacles wearer.

The input unit may request the inputs of the full corrected value and the value of the prescription power of the first refractive unit. The blur sensitivity estimating unit estimates the blur sensitivity based on a relation between the full corrected value transmitted by the transmission unit and the value of the prescription power of the first refractive unit transmitted by the transmission unit.

The input unit may request an input of the visual acuity obtained by using the prescription power of the first refractive unit. The first area adjusting unit adjusts the area of the clear-visible region of the first refractive unit based on the blur sensitivity estimated by the blur sensitivity estimating unit and the visual acuity obtained by using the prescription power of the first refractive unit transmitted by the transmission unit.

A method for manufacturing a progressive lens according to the present embodiment includes a blur sensitivity estimating step for estimating a blur sensitivity indicating a sensitivity of the spectacles wearer relative to a blur at the time when the spectacles wearer looks at an object through lenses having the prescription power of the first refractive unit, a first area adjusting step for adjusting the area of the clear-visible region of the first refractive unit based on the blur sensitivity estimated in the blur sensitivity estimating step, a design reference lens selecting step for selecting at least a single basic design lens for coinciding with or approximating to the lens of which the area of the first refractive unit is adjusted in the first area adjusting step from among a predetermined basic design lens group as a design reference lens of the progressive lens to be worn by the spectacles wearer, and a data creating step for creating data to manufacture the progressive lens based on a lens design of the design reference lens selected in the design reference lens selecting step. The progressive lens includes the first refractive unit having a first refractive power, a second refractive unit having a second refractive power in which a refractive power (addition power) is applied to the first refractive power for near vision, and a corridor in which the refractive power is progressively changed along a meridian from the first refractive unit to the second refractive unit.

A manufacturing method for a progressive lens according to the present embodiment includes a step for manufacturing a progressive lens by using the data created by performing the method for manufacturing the progressive lens.

A program according to the present embodiment is to make a computer execute the method for manufacturing the progressive lens.

According to the present embodiment, a method, a program, and a device for manufacturing a progressive lens, a manufacturing method for the progressive lens, and a lens supply system which are suitable for reducing dissatisfaction of an expected wearer relative to a vision through the progressive lens are provided.

Brief Description of Drawings

Fig. 1 is a block diagram of a structure of a spectacle lens manufacturing system according to an embodiment of the present invention.
Figs. 2A to 2D are diagrams of exemplary screens of a spectacle selector application displayed on a shop computer provided in a spectacles store according to the embodiment of the present invention.
Fig. 3 is a diagram of a flowchart of a manufacturing process for a spectacle lens in a spectacle lens manufacturing plant according to the embodiment of the present invention.
Fig. 4 is a diagram of a flow for selecting a progressive addition lens by using the spectacle selector application according to the embodiment of the present invention.
Fig. 5 is a diagram of a basic design lens group (power distribution (average power error and astigmatism)) in which balances between distance portions and near portions are arranged by type.
Figs. 6A to 6D are diagrams to describe an effect obtained by introducing an eighth basic design point DES8 as a design point of the progressive addition lens.
Fig. 7 is a graph of an entropy of a subject (expected wearer) according to sharpness of a presented image.
Fig. 8 is a graph of the entropy of the subject (expected wearer) according to the sharpness of the presented image.
Fig. 9 is a graph of the entropy of the subject (expected wearer) according to the sharpness of the presented image.

Description of Embodiments

A spectacle lens manufacturing system according to an embodiment of the present invention is described below with reference to the drawings.

Spectacle lens manufacturing system 1

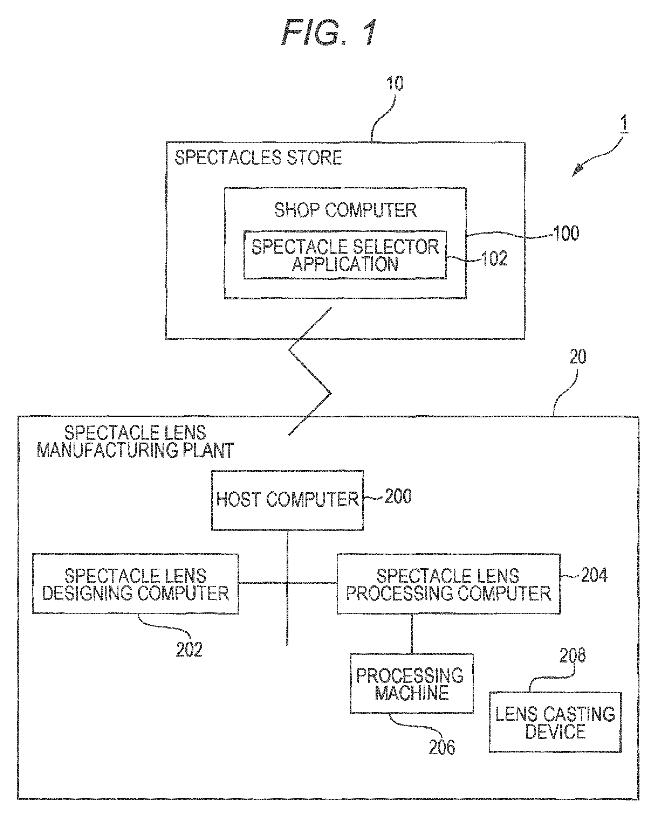

Fig. 1 is a block diagram of a structure of a spectacle lens manufacturing system 1 according to the present embodiment. As illustrated in Fig. 1, the spectacle lens manufacturing system 1 includes a spectacles store 10 for ordering spectacle lenses according to a prescription to a customer (expected wearer) and a spectacle lens manufacturing plant 20 for manufacturing the spectacle lenses after receiving the order from the spectacles store 10. The order to the spectacle lens manufacturing plant 20 is performed via data transmission via a predetermined network such as Internet and FAX. An ophthalmologist and a general consumer may be included in the orderer.

Spectacles store 10

A shop computer 100 is provided in the spectacles store 10. The shop computer 100 is, for example, a tablet terminal, a smartphone, a desktop personal computer (PC), and a notebook PC. An application 102 for selecting spectacles suitable for the expected wearer (referred to as "spectacle selector application" below) is installed to the shop computer 100.

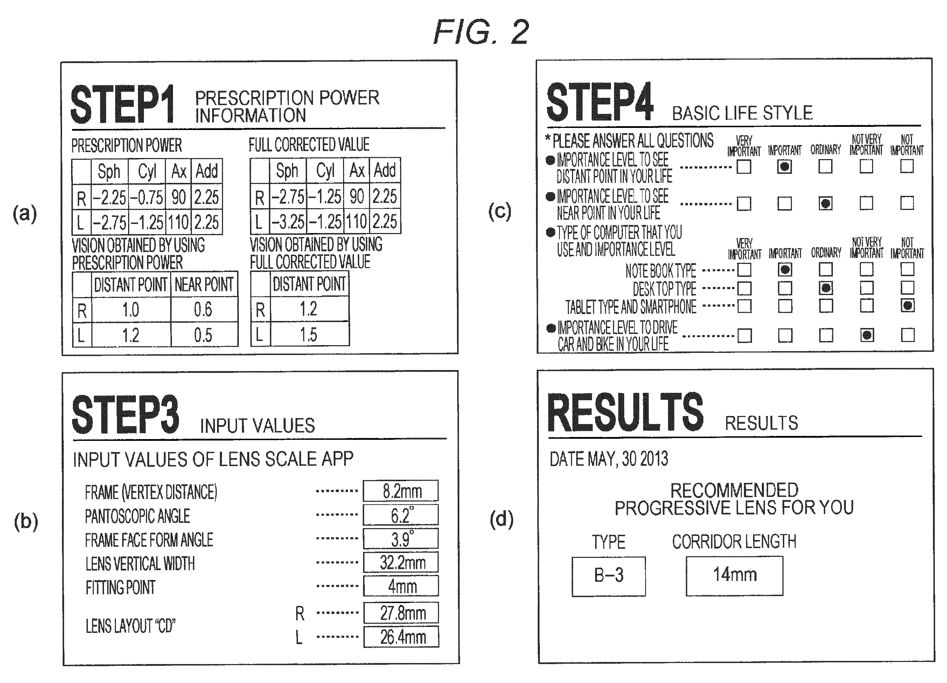

Figs. 2A to 2D are exemplary screens of the spectacle selector application 102 displayed on the shop computer 100. Figs. 2A and 2B are exemplary input screens of lens data and frame data. For example, the lens data includes a prescription value (spherical power, cylindrical power, cylinder axis, prismatic addition power, prism base direction, addition power, pupillary distance (PD)), a lens material, a refractive index, a kind of an optical design, an outer diameter of a lens, the thickness of the lens, the edge thickness, decentration, a base curve, wearing conditions of the spectacle lens (vertex distance, pantoscopic angle, and frame face form angle), a kind of the spectacle lens (single vision spherical, single vision aspherical, multifocal (bifocal and progressive), coating (hard coating, antireflection coating, ultraviolet cutting, and the like), color tinting), and layout data according to the request of the expected wearer. The frame data includes frame shape data selected by the expected wearer. Also, as illustrated in Fig. 2A, information obtaining items of the spectacle selector application include the full corrected value (spherical power, cylindrical power, cylinder axis, and addition power), distance visual acuities and near visual acuities of right and left eyes by the prescription power, and visual acuities of the right and left eyes by the full corrected value in addition to the general lens data and frame data.

Fig. 2C is an exemplary screen for obtaining information on a life style of the expected wearer. This information obtaining item includes, for example, the basic life style of the expected wearer (having a work that is a desk work and driving a car for a long time) and a life scene (what the expected wearer do inside or outside the room).

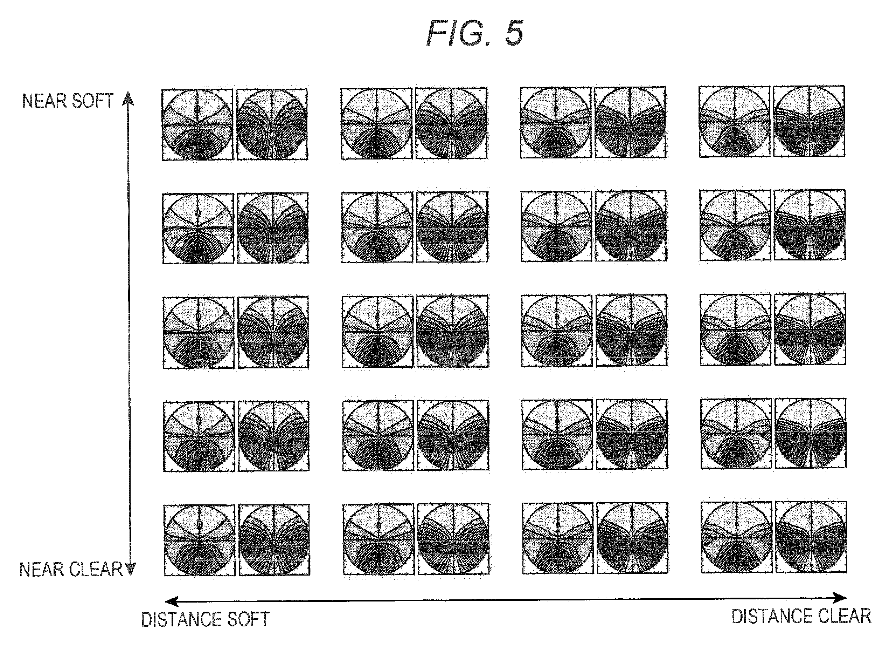

An inspector performs an optometric work by using a trial lens and the like. Next, the inspector or the expected wearer inputs data to the screen such as an input screen for various data and the screen inquiring the life style displayed on the shop computer 100 in the spectacles store 10. When receiving all the inputs to all the screens necessary for designing the spectacle lens, the shop computer 100 selects at least a single basic design lens from among the predetermined basic design lens group (refer to Fig. 5 and to be described in detail below) and displays the selected basic design lens on the screen (refer to Fig. 2D). When the basic design lenses displayed on the screen have been selected as the spectacle lenses to be worn by the expected wearer, the shop computer 100 transmits ordering data (lens data and frame data) to the spectacle lens manufacturing plant 20, for example, via the Internet.

The spectacle selector application 102 may be installed to a server (not shown) provided on the network. In this case, for example, the spectacle selector application 102 is used on the server by using a general browser of the shop computer 100 as a graphical user interface (GUI) or via an exclusive GUI application installed to the shop computer 100. Communication between the shop computer 100 and the server is sequentially performed at the timing when the screen such as the input screen for various data and the screen inquiring the life style displayed on the browser (screen downloaded from the server (spectacle selector application 102) via the network) is changed. The ordering data is transmitted from the server to the spectacle lens manufacturing plant 20 at the time when the basic design lenses displayed on the browser and the like have been selected as the spectacle lenses to be worn by the expected wearer.

Spectacle lens manufacturing plant 20

In the spectacle lens manufacturing plant 20, a local area network (LAN) having a host computer 200 provided at the center is constructed. A number of terminal devices including a spectacle lens designing computer 202 and a spectacle lens processing computer 204 are connected to the LAN. The spectacle lens designing computer 202 and the spectacle lens processing computer 204 are general PCs, and a program for designing the spectacle lens and a program for processing the spectacle lens are installed to the respective computers. The ordering data transmitted from the shop computer 100 via the Internet is input to the host computer 200. The host computer 200 transmits the input ordering data to the spectacle lens designing computer 202.

Manufacturing spectacle lens in spectacle lens manufacturing plant 20

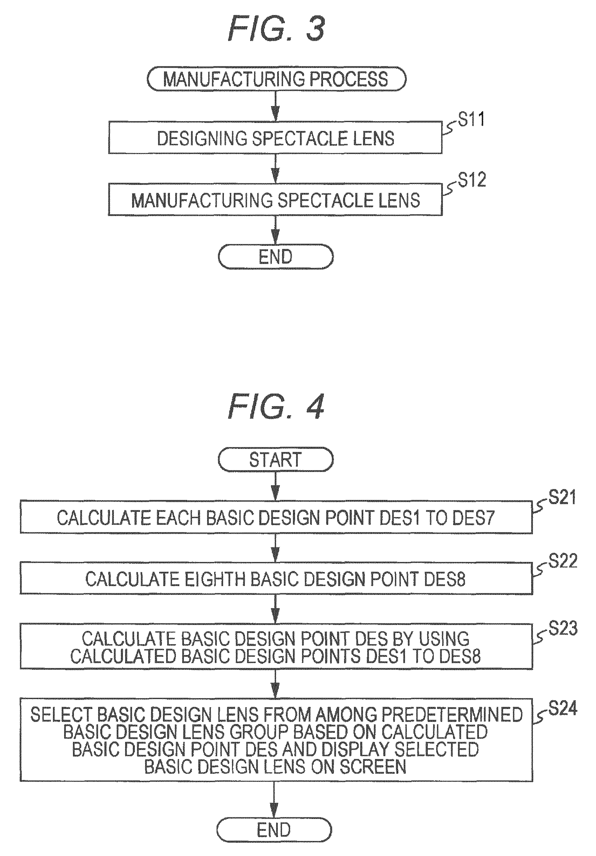

S11 in Fig. 3 (designing spectacle lens)

Fig. 3 is a flowchart of a manufacturing process for the spectacle lens in the spectacle lens manufacturing plant 20. A program for designing the spectacle lens according to the order is installed to the spectacle lens designing computer 202. The spectacle lens designing computer 202 creates lens design data based on the ordering data (lens data) and creates lens shape processing data based on the ordering data (frame data) . The spectacle lens designing computer 202 forwards the created lens design data and lens shape processing data to the spectacle lens processing computer 204.

S12 in Fig. 3 (manufacturing spectacle lens)

The spectacle lens processing computer 204 reads the lens design data and the lens shape processing data forwarded from the spectacle lens designing computer 202 and drives and controls a processing machine 206.

For example, a case is considered where a plastic spectacle lens is manufactured by using a cast polymerization method. In this case, the processing machine 206 produces molding dies corresponding to both surfaces including an outer surface (convex) and an inner surface (concave) of the lens by grinding and polishing a material such as metal, glass, and ceramic based on the lens design data. A pair of produced molding dies is arranged opposed to each other with an interval corresponding to the thickness of the spectacle lens, and an adhesive tape is wound around an outer peripheral surface of each molding die. A space between the molding dies is sealed. When the pair of molding dies is set to the lens casting device 208, a hole is provided in a part of the adhesive tape, and liquid monomer is injected into a cavity (sealed space between molding dies) through the hole. The liquid monomer injected and filled into the cavity is cured by heat and ultraviolet ray irradiation. According to this, a spectacle lens base material can be obtained to which a transferred surface shape of the pair of molding dies and a circumferential shape formed by the adhesive tape are transferred. The spectacle lens base material obtained by curing it is removed from the molding die. A residual stress of the spectacle lens base material separated from the die is removed by annealing. After that, various kinds of coating such as dyeing, hard coating, an antireflection film, and ultraviolet cutting is performed. Accordingly, the spectacle lens is completed and is delivered to the spectacles store 10.

Also, in order to improve productivity, a semifinished lens blank group may be previously prepared in the spectacle lens manufacturing plant 20 for the order of the spectacle lens. In the semifinished lens blank group, powers of the lenses over the entire produce range are divided into groups, and the lens has a convex curve shape (for example, spherical shape and aspherical shape) and a lens diameter suitable for the power range of each group. For example, the semifinished lens blank is a resin blank or a glass blank, and the convex and the concave are respectively an optical surface (completed surface) and a non-optical surface (uncompleted surface). In this case, an optimal semifinished lens blank is selected based on the lens data, and the selected semifinished lens blank is set to the processing machine 206. The processing machine 206 produces an uncut lens by grinding and polishing the concave of the set semifinished lens blank based on the lens design data. Various kinds of coating such as dyeing, hard coating, an antiref lection film, and ultraviolet cutting is performed to the uncut lens of which the concave shape has been produced. Periphery machining is performed to the outer peripheral surface of the uncut lens, to which various kinds of coating has been performed, based the lens shape processing data created by the spectacle lens designing computer 202. The spectacle lens processed in a lens shape is delivered to the spectacles store 10. Also, in the spectacle lens manufacturing plant 20, the spectacle lens may be manufactured and processed by using a lens blank of which both the convex and the concave are formed in spherical shapes instead of the semifinished lens blank.

Description on spectacle lens to be designed and manufactured

In the present embodiment, a spectacle lens having a region where refractive power is progressively changed between two reference points and a spectacle lens having a similar region (referred to as "progressive lens" below as a word including these spectacle lenses) are designed and manufactured. For example, the progressive lens designed and manufactured in the present embodiment includes a plurality of lenses such as a progressive lens, a middle-near distance progressive lens, and a near distance degressive lens according to the uses. Also, a plurality of design types of progressive refractive elements exist. For example, the design type of the progressive refractive element includes a one surface progressive type for applying the progressive refractive element to the convex (object side surface) or the concave (eyeball side surface), a double-sided progressive type for allocating the progressive refractive element to the convex and the concave, and a double-sided composite type for allocating the progressive refractive element in the vertical direction to the convex and the progressive refractive element in the horizontal direction to the concave.

For example, the progressive addition lens covers a wide distance range from a long distance to a short distance, and the progressive addition lens is designed basically for distance vision. Specifically, in the progressive addition lens, a distant vision region (distance portion) is widely laid out in a region above the center of the lens, and a near vision region (near portion) is laid out in a limited region in a lower part of the lens. An intermediate vision region (corridor) for connecting the reference point on the side of the distance portion to the reference point on the side of the near portion is a region where the refractive power progressively changes between the two reference points. The width of an area of clear vision along a meridian is narrower than that of the distance portion and that of the near portion.

For the expected wearer for wearing the progressive addition lens, it is preferable that all the regions including the distance portion, the near portion, and the corridor be wide. However, regarding the progressive addition lens having the structure in which the refractive power changes in the lens, it is technically impossible to widely ensure all the regions. The progressive addition lens has characteristics such that the wider the distance portion and the near portion are laid out and the shorter the distance between the distance portion and the near portion (corridor length) is, the narrower the width of the area of clear vision gets by the generation of unnecessary astigmatism on both sides of the corridor. Therefore, in the region on both sides of the corridor, the object cannot be clearly looked, and the object is looked distorted and with swaying.

In this way, regarding the progressive addition lens, when the distance portion and the near portion are unnecessarily enlarged, the unnecessary astigmatism generated on the both sides of the corridor gets stronger, and the object is looked strongly distorted and with swaying. Therefore, in the progressive addition lens, the distance portion is comparatively laid out to be wide, and the near portion is laid out to be narrow to a certain extent. Accordingly, the unnecessary astigmatism, distortion and swaying effect generated in the region on the both sides of the corridor are reduced.

Variety of the sizes of the distance portion and near portion are prepared according to the usage of the progressive addition lens and the life style of the expected wearer. As the specific variation, there are a type for reducing the unnecessary astigmatism, distortion and swaying effect generated on the both sides of the corridor by narrowing the clear-visible region of the distance portion than the standard design, a type for widening the clear-visible region of the distance portion from the standard design, a type for reducing the unnecessary astigmatism, distortion and swaying effect generated on the both sides of the corridor by narrowing the clear-visible region of the near portion than the standard design, and a type for widening the clear-visible region of the near portion from the standard design.

The middle-near distance progressive lens is designed for the intermediate vision compared with the progressive addition lens and covers all the distances regarding the indoor works such as PC work, other desk work, and housework. The near distance degressive is designed for the near vision and covers a distance, for example, from the region around the hand to the depth of the desk regarding the PC work and other desk work. The middle-near distance progressive lens and the near distance degressive addition lens have the similar characteristics to the progressive addition lens. Variety of types of middle-near distance progressive lenses and the near distance degressive lenses are prepared according to the usage and the life style of the expected wearer.

In the embodiment below, the description on the progressive addition lens is made. However, a content in which the progressive addition lens is replaced with the other progressive lens such as the middle-near distance progressive lens and the near distance degressive lens is included in the embodiment of the present invention.

Description on vision of object through spectacle lens

A way a person perceives a blur image and a sharp image (blur sensitivity) is different for each person, and the blur sensitivity is determined based on the quality of the perceived image. It is considered that the quality of the perceived image is determined according to a value of the prescription power of the spectacle lens, the size of a pupil diameter, transparency of an ocular media in an eyeball, and a perceived image processed and formed in the brain.

An ability to form the perceived image in the brain is different for each person. For example, a case is considered where a person looks at a slightly blur image. In this case, the image that the person looks is improved by the process in the brain and is perceived as an image with no blur. Also, the image is not improved by the process in the brain and is perceived as the image with the blur.

Visual acuity of expected wearer

Information including a visual acuity level of the expected wearer by using the prescription power of the spectacle lens measured and determined by the optometry is not provided to a spectacle lens maker. The information is obtained by the ophthalmologist or optometrist, the person who performs the optometry, or in the spectacles store. However, the progressive addition lens has characteristics such that the size of each region (distance portion, corridor, and near portion) is restricted and the aberration amount generated around the region is changed according to the size of each region. When the progressive addition lens is newly created, a basic design of the progressive addition lens to be selected is different according to whether the visual acuity level of the expected wearer is high. For example, when the high far visual acuity can be obtained by using the determined prescription power, the expected wearer hardly feels an inconvenience even when the expected wearer wears the progressive addition lens having some degree of aberration generated around the distance portion. On the other hand, when the far visual acuity cannot be obtained enough by using the determined prescription power, the expected wearer easily perceives the blur caused by the aberration and feels disturbed and unsatisfied in a case where the expected wearer wears the above progressive addition lens (having some degree of aberration generated around the distance portion).

In this way, even when the expected wearer wears the spectacles having the same progressive addition lenses, the feeling of the expected wearer relative to the aberration allocated in the progressive addition lens is different according to the visual acuity level of the expected wearer.

Prescription power determined by using existing method

As described above, the optometric work (measuring and determining prescription power) by the inspector is performed in a form in which the inspector asks the expected wearer about the visual acuity of the expected wearer relative to the object through trial lens and the expected wearer responds to it. However, when the power of the trial lens reaches the power close to the optimal power, the expected wearer sometimes feels difficulty to determine which one of the trial lenses having the power close to the optimal power provides better vision and does not know how to respond. That is, the response of the expected wearer includes some degree of inaccuracy.

Also, as described above, the prescription power determined by the optometric work may be adjusted according to the request of the expected wearer and the experimental judgment of the inspector. However, when the progressive addition lens is ordered, regarding the power, the value of the prescription power finally determined in the spectacles store and the like is informed to the spectacle lens maker. The information on the kind of the prescription power (for example, the prescription power is the full corrected value or how many adjustment has been added to the full corrected value) is not informed. In this way, the spectacle lens maker has no information on how the prescription power has been adjusted from the full corrected value. Therefore, the spectacle lens maker cannot acquire the vision of the expected wearer when the expected wearer wears the lenses such as aspherical lenses and progressive lenses in which the refractive power is changed over the lenses.

Blur sensitivity

The inventor of the present invention has considered that the expected wearer is sometimes unsatisfied about the vision of the expected wearer relative to the object by using the spectacle lenses manufactured in a state where the maker cannot acquire the above information.

For example, a case is considered where an aspherical design lens with a single vision is ordered as a spectacle lens having a negative power to correct near sight. When the ordered prescription power is the full corrected value and improves the visual acuity level of the expected wearer to the high level, the aberration is improved by the aspherical design. Therefore, the blur is reduced than that in a case where a spherical design lens is used. On the other hand, for example, when the ordered prescription power has been adjusted from the full corrected value to the side of a positive power (adjustment to reduce negative power has been performed), growth of the average refractive power around the lens is reduced according to the improvement of the aberration around the lens by the aspherical design. Therefore, when the expected wearer looks at a distant point via the periphery of the lens, the image with blur is rather obtained. When using the prescription power with the reduced negative power, the spherical design lens in which the aberration is not corrected and the average refractive power in the periphery of the lens gets stronger on the negative side may provide a clear image when the expected wearer looks at the distant point via the periphery of the lens. In consideration of this case, it is considered that the spectacle lens capable of reducing dissatisfaction of the expected wearer can be designed when the kind of the prescription power is fed back to the lens design.

Also, the above idea can be applied to the progressive addition lens. For example, when the distant vision is not very important for the expected wearer (person who does not need to drive and has the life style for mainly acting in the room), the necessity to secure the wide distance portion is low. In order to reduce the aberration generated on the both sides of the corridor and to reduce the generation of the distortion and swaying of the image which is a specific fault of the progressive addition lens, the design is preferable in which the width of the clear corridor is widened instead of narrowing the distance portion and the astigmatism and a power error in the periphery of the lens are reduced.

In a case of the progressive addition lens in which the distance portion is designed to be slightly narrower, the refractive power close to the prescription power is allocated in the upper center region (center region of distance portion) of the lens. Therefore, when the person looks at the distant point through the center region of the distance portion, the person can clearly look at the object. On the other hand, in a peripheral region of the distance portion shifted from the upper center of the lens to the side of the nose or ear, the aberration (astigmatism and power error to the side of positive power) is generated. Therefore, in a case where the person looks at the distant point through the peripheral region of the distance portion, the person looks at the object with the blur since the refractive power is different from the prescription power by the generated aberration even when the ordered prescription power is the full corrected value. However, the basic visual distance of the person having the life style for mainly acting in the room is limited to a distance in the room. Therefore, even when the person looks at the distant point in the room through the peripheral region of the distance portion, the person can clearly look at the object, and the aberration does not cause the problem.

However, when the ordered prescription power has been adjusted to the side of the positive power relative to the full corrected value, the person looks at the object with blur by the adjusted power in a case where the person looks at the distant point through the center region of the distance portion. When the person looks at the distant point through the peripheral region of the distance portion, the person looks at the object with more blur since the aberration allocated to the peripheral region (especially, power error to the side of the positive power) and the aberration for the adjusted power are superimposed. When the person looks at the distant point through the peripheral region of the distance portion, there is a possibility that the person looks at the object with the blur even when looking at the distant point in the room. To solve the above problem, for example, to design the wider distance portion can be considered. Accordingly, a difference between the vision at the time when the person looks at the distant point in the room through the center region of the distance portion and the vision at the time when the person looks at the distant point in the room through the peripheral region of the distance portion is reduced. Therefore, the person hardly recognizes the blur in the vision through the peripheral region of the distance portion.

According to the above, it is considered that the relation between the full corrected value determined by the optometric work and the value of the prescription power adjusted and determined by the optometric work is closely related to the blur sensitivity perceived by the expected wearer when the expected wearer looks at the object through the progressive addition lenses. As a result of intensive studies, the inventor of the present invention obtains knowledge such that the blur sensitivity of the expected wearer can be estimated, that is, the level of the blur sensitivity can be estimated based on the relation (difference) between the full corrected value determined by the optometric work and the value of the prescription power adjusted and determined by the optometric work, and the appropriate design of the progressive addition lens can be determined based on the estimated blur sensitivity. Also, the inventor obtains knowledge such that more appropriate design of the progressive addition lens can be determined in consideration of the vision level of the expected wearer.

Method for selecting progressive addition lens by using spectacle selector application 102

Fig. 4 is a flow for selecting the progressive addition lens with a reference design suitable for the expected wearer from among the basic design lens group which has been previously prepared as design variety by using the spectacle selector application 102.

S21 in Fig. 4 (calculation of each basic design point DES1 to DES7)

In a processing step S21, basic design points DES1 to DES7 are calculated. Here, outlines of the basic design points DES1 to DES7 are described. Detailed description on the basic design points DES1 to DES7 can be obtained by referring to JP 5094968 B1, USB08690323.

The spectacle selector application 102 calculates an average value MPW of the right and left eyes based on distance spherical powers SPH, cylindrical powers CYL, and cylinder axis AX of the right and left eyes input via an input screen for various data displayed on the shop computer 100. Then, the spectacle selector application 102 calculates the first basic design point DES1 based on the calculated average value MPW.

The spectacle selector application 102 calculates the second basic design point DES2 based on right and left addition powers ADD input via the input screen for the various data displayed on the shop computer 100.

The spectacle selector application 102 calculates the third basic design selection point DES3 based on frame information (pantoscopic angle PA of lens) input via the input screen for various data displayed on the shop computer 100.

The spectacle selector application 102 calculates an average distance between the apices MVD based on the frame information input via the input screen for the various data displayed on the shop computer 100 (distances VDL and VDR between right and left lens rear surfaces and right and left eyeball corneal apices) and calculates the fourth basic design selection point DES4 based on the calculated average distance between the apices MVD.

The spectacle selector application 102 calculates the fifth basic design selection point DES5 based on importance levels A1 to A6 regarding each life scene (TV watching, PC, playing instruments, cooking, gardening, dancing and fitness), importance levels B1 to B6 regarding each life scene (shopping, dinner and party, travel and resort, taking landscape photograph, running, and walking), and importance levels C1 to C6 regarding each life scene (driving, touring, watching sports in stadium, golf, climbing and hiking, theater and movie) input via the screen for inquiring the life style displayed on the shop computer 100.

The spectacle selector application 102 calculates the sixth basic design selection point DES6 based on significance FWT (importance level relative to distant vision of expected wearer) input via the input screen for the various data displayed on the shop computer 100.

The spectacle selector application 102 calculates the seventh basic design selection point DES7 based on type information KBtp (information on the kind of lenses of spectacles, which has been worn by the expected wearer last time, such as progressive lens, multifocal lens, and short focus lens), the degree of satisfaction SAT of the spectacles which have been used last time, and average values MPWp and MPW input via the input screen for the various data displayed on the shop computer 100.

S22 in Fig. 4 (calculation of eighth basic design point DES8)

In a processing step S22, an eighth basic design point DES8 is calculated.

S23 in Fig. 4 (calculation of basic design point DES)

In a processing step S23, a basic design point DES is calculated based on the DESTT (= DES1 + DES2 + DES3 + DES4 + DES5 + DES6 + DES7 + DES8), CURD (index of spectacles type which has been used last time), and the degree of satisfaction SAT. Details of the calculation of the basic design point DES (content except for eighth basic design point DES8) can be obtained by referring to JP 5094968 B1, USB08690323.

S24 in Fig. 4 (selection of basic design lens)

In a processing step S24, at least a single basic design lens is selected from among the predetermined basic design lens group as the design reference lens for the progressive addition lens to be worn by the expected wearer based on the basic design point DES calculated in the processing step S23 (calculation of basic design point DES) and is displayed in the screen on the shop computer 100.

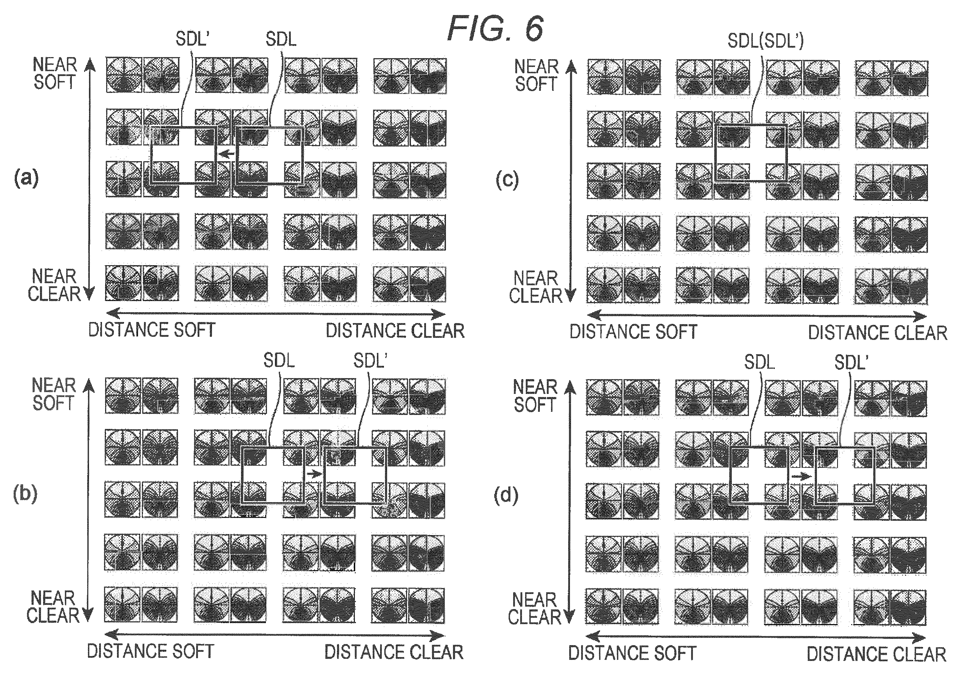

Here, Figs. 5 and 6A to 6D are diagrams to describe an effect obtained by introducing the eighth basic design point DES8 as the design point of the progressive addition lens.

Fig. 5 is a diagram of a basic design lens group (power distribution (average power error and astigmatism)) in which balances between the distance portions and the near portions are arranged by type. In Fig. 5, the basic design lens (power distribution) on the right side is a distance clear type (with smaller aberration in peripheral region of distant vision region) lens having a wider distance portion, and the basic design lens (power distribution) on the left side is a distance soft type (with larger aberration in peripheral region of distant vision region) lens having a narrower distance portion. Also, in Fig. 5, the basic design lens (power distribution) on the lower side is a near clear type (refractive power for near vision is widely allocated) lens having a wider near portion. The basic design lens (power distribution) on the upper side is a near soft type (refractive power for near vision is narrowly allocated) lens having a narrower near portion. The allocations of the basic design lenses arranged in Fig. 5 (and Figs. 6A to 6D to be described) are basic allocations before performing inset to move the near portion to the nose side. When it is determined that the basic design allocation is used for the right lens or the left lens, the inset to move the near portion to the nose side is performed.

The design of the distance clear type is mainly suitable for the expected wearer having a condition in which a slight blur of the image in the distance portion (or near portion) is easily concerned (driving at high frequency and the like). Compared with the standard type (in the present embodiment, lens type arranged at the center of basic design lens group), the clear-visible region in the corridor is narrowed. Instead of that, the clear-visible region in the distance portion (or near portion) is widely secured. According to the design of the distance clear type, although the unnecessary astigmatism, warp, and jump generated in the regions on the both sides of the corridor are increased, the clear-visible region in the distance portion (or near portion) is widened. Therefore, the design of the distance clear type is suitable for an expected wearer who puts much value on the distant vision (or near vision).

The design of the distance soft type is mainly suitable for the expected wearer having a condition in which a slight blur of the image in the distance portion (or near portion) is hardly concerned (do not drive and the like). In the design of the distance soft type, the clear-visible region in the distance portion (or near portion) is narrowed. Instead of that, the corridor is widely secured. According to the design of the distance soft type, although the clear-visible region in the distance portion (or near portion) is narrowed, the unnecessary astigmatism, warp, and jump generated in the regions on the both sides of the corridor are reduced. Therefore, the design of the distance soft type is suitable for an expected wearer who puts much value on the intermediate vision.

Four examples of a case where a design type (distance clear type, distance soft type, and the like) of the distance portion is changed based on the eighth basic design point DES8 are described with reference to Figs. 6A to 6D. In Figs. 6A to 6D, for convenience of description, a range of the basic design lens selected based on the basic design point DES at the time when the DESTT is an addition value of the basic design points DES1 to DES7 (value to which eighth basic design point DES8 is not added) is denoted with a reference numeral SDL. Also, a range of the basic design lens selected based on the basic design point DES at the time when the DESTT is an addition value of the basic design points DES1 to DES8 is denoted with a reference numeral SDL'. In Figs. 6A to 6D, the basic design lens (power distribution (average power error and astigmatism)) partially included in the range SDL' is selected as the design reference lens of the progressive addition lens to be worn by the expected wearer.

Also, in the cases 1 to 4 below, it is determined whether the visual acuity level of the expected wearer through the distance portion is high. Specifically, when a both-eye vision value VAb measured by the optometric work is equal to or more than 0.9, it is determined that the vision level is high. When the both-eye vision value VAb is less than 0.9, it is determined that the vision level is not high. When the both-eye vision value VAb is not measured, the both-eye vision value VAb is calculated based on the visions of right and left eyes (far visions of right and left eyes obtained by using prescription power input via screen in Fig. 2A). The following formula is an exemplary calculation of the both-eye vision value VAb.

```
both-eye vision value VAb = ((vision of right eye + vision of

left eye))/2) × 1.1
```

Case 1: visual acuity level of expected wearer through distance portion is high and distance prescription power is full corrected value or value approximating to it

Since a distance prescription power is the full corrected value or a value approximating to it, the blur in the image of the object when the person looks at the distant point is small. The relation (difference) between the distance prescription power and the full corrected value is obtained based on the prescription power and the full corrected value input via the screen in Fig. 2A. Therefore, even when the design type of the distance portion is slightly changed, the blur is hardly perceived at least when the person looks at the distant point. Therefore, in case 1, it is estimated that the expected wearer is dull relative to the blur when using the distance prescription power and has low blur sensitivity. In addition, it is estimated that the blur sensitivity gets lower as the distance prescription power and the full corrected value are closer to each other. When the distance prescription power adjusted to the negative side from the complete correction is prescribed, although this is not normally performed on purpose, it is estimated that the blur sensitivity is low.

In case 1, the eighth basic design point DES8 is calculated according to the following formula.

$$DES8 = (\Delta PW - 0.25) \times (VAb - 0.6) \times 30$$

$\Delta PW$ is the average value of a difference between average powers of the distance prescription powers (right) and full corrected values (right) and a difference between average powers of distance prescription powers (left) and full corrected value (left).

The formula representing $\Delta PW$ is as follows. $\Delta PW = ((SPH_R + CYL_R/2) - (SPH_{R0} + CYL_{R0}/2) + (SPH_L + CYL_L/2) - (SPH_{L0} + CYL_{L0}/2))/2$.

When the eighth basic design point DES8 is a negative value, this causes an effect to select a soft design for the distance portion. When the eighth basic design point DES8 is a positive value, this causes an effect to select a clear design for the distance portion. In case 1, the eighth basic design point DES8 is a negative value. Therefore, as illustrated in Fig. 6A, the range of the selected basic design lens is shifted from the range SDL to the range SDL' on the distance soft side. The amount of the shift is larger as the absolute value of the eighth basic design point DES8 gets larger. The amount of the shift is smaller as the absolute value of the eighth basic design point DES8 gets smaller.

In case 1, the visual acuity level of the expected wearer through the distance portion is high. Therefore, even when the range of the selected basic design lens is shifted to the distance soft side (that is, side where blur increases), this does not cause a real problem.

Case 2: visual acuity of expected wearer through distance portion is high and distance prescription power is adjusted to positive power from full corrected value

Since the distance prescription power is adjusted to a value apart from the full corrected value to the positive power side, the blur is slightly larger when the person looks at the distant point. Especially, the blur generated when the person looks at the object through the peripheral region of the distance portion is large. Therefore, for example, when the design type of the distance portion is changed to the further positive power side, there is a possibility that the blur at the time of looking at the distant point increases. Therefore, in case 2, it is estimated that the expected wearer is sensitive relative to the blur when using the distance prescription power and has high blur sensitivity. In addition, it is estimated that the blur sensitivity gets higher as the distance prescription power and the full corrected value are deviated from each other.

Also, in case 2, the eighth basic design point DES8 is calculated by using the same formula as in case 1. In case 2, the eighth basic design point DES8 is a positive value. Therefore, as illustrated in Fig. 6B, the range of the selected basic design lens is shifted from the range SDL to the range SDL' on the distance clear side (side close to full corrected value). The amount of the shift is larger as the absolute value of the eighth basic design point DES8 gets larger. The amount of the shift is smaller as the absolute value of the eighth basic design point DES8 gets smaller.

In case 2, although the visual acuity level of the expected wearer through the distance portion is high, it is considered that the closer to the full corrected value the value is, the higher the visual acuity level is.

Case 3 : visual acuity of expected wearer through distance portion is not high and distance prescription power is full corrected value or value approximating to it

In case 3, similarly to case 1, since the distance prescription power is the full corrected value or the value approximating to it, it is estimated that the expected wearer has low blur sensitivity. In case 3, the eighth basic design point DES8 is calculated by using the following formula to consider a case where it is determined that the vision level is not high.

$$DES8 = \Delta PW \times 10$$

In case 3, the shift to the distance soft side can be performed similarly to case 1. However, since the visual acuity level of the expected wearer through the distance portion is not high, the shift of the range of the basic design lens is not recommended. Therefore, as illustrated in Fig. 6C, the range of the selected basic design lens is not shifted to the distance soft side.

Case 4: visual acuity level of expected wearer through distance portion is not high and distance prescription power is adjusted to positive power side from full corrected value

In case 4, similarly to case 2, since the distance prescription power is adjusted to a value apart from the full corrected value to the positive power side, it is estimated that the expected wearer has high blur sensitivity. Since the visual acuity level is not high, the calculation formula of the eighth basic design point DES8 is the same as that in case 3.

In case 4 , although the visual acuity level of the expected wearer through the distance portion is not high, it is considered that the visual acuity level is improved when the value is adjusted to be closer to the full corrected value. Therefore, as illustrated in Fig. 6D, the range of the selected basic design lens is shifted from the range SDL to the range SDL' on the distance clear side (side close to full corrected value). The amount of the shift is larger as the absolute value of the eighth basic design point DES8 gets larger. The amount of the shift is smaller as the absolute value of the eighth basic design point DES8 gets smaller.

Creation of ordering data

When the single basic design lens is selected from among the basic design lens selected in the processing step S24 in Fig. 4 (selection of basic design lens of progressive addition lens to be worn), the spectacle selector application 102 creates the ordering data (lens data and frame data) of the progressive addition lens suitable for the physical feature of the expected wearer, a usage condition, and the prescription power based on the selected basic design lens and transmits the data to the spectacle lens manufacturing plant 20.

Change of design type (near clear type, near soft type, and the like) of near portion

In the above description, the design type of the distance portion has been changed. However, the design type of the near portion may be also changed. For example, as described below, it is preferable that the design type of the near portion be changed based on the near vision of both eyes measured by using a near vision chart.

As the near vision chart used to measure the near vision, a vision chart to measure the near visual acuity using printed matters and a vision chart displayed on a compact display device placed in a short distance are exemplified. The near visual acuity is normally measured at a distance of 40 cm. However, when the distance for near vision of the expected wearer is definite, it is preferable to measure the near vision at the distance. Three cases about the change of the design type of the near portion according to the near visual acuity (near both-eye visual acuity value VAbn) are described.

$$\text{Case of } 0.6 \leq \text{VAbn} < 1.0$$

In this case, since a near vision of a normal level can be obtained, it is not necessary to change the design type of the near portion in consideration of the near vision. The design type of the near portion is mainly determined based on information other than the near vision such as the life scene of the expected wearer (for example, whether the expected wearer often reads books and newspapers).

$$\text{Case of } 1.0 \leq \text{VAbn}$$

In this case, an especially good near vision can be obtained. Since it is considered that the near vision through the near portion is comfortably obtained, the near soft type having slightly narrower near portion according to the value of the near visual acuity relative to the design type of the near portion based on the information other than the near visual acuity is selected (for example, in Fig. 6A, range SDL is shifted upward). In the near soft type, the clear region in the corridor is widened instead of narrowing the near portion, and the unnecessary astigmatism, distortion, and swaying effect generated in the regions on the both sides of the corridor are reduced. The range to narrow the width of the near portion of the progressive lens is limited. In this case, it is considered that the range to narrow the width of the near portion is limited to about 20% relative to the standard design regardless of the both-eye visual acuity value VAbn.

$$\text{Case of } \text{VAbn} < 0.6$$

In this case, a high near visual acuity is not obtained. In order to make the person comfortable regarding the near vision through the near portion, a near clear type having a near portion wider than that of the design type of the near portion based on the information other than the near visual acuity according to the value of the near visual acuity is selected (for example, range SDL is shifted downward in Fig. 6A). However, the clear region in the corridor is narrowed instead of widening the near portion, and the unnecessary astigmatism, distortion, and swaying effect generated in the regions on the both sides of the corridor are increased. The range to widen the width of the near portion of the progressive lens is limited. In this case, it is considered that the range to widen the width of the near portion is limited to about 30% relative to the standard design regardless of the both-eye visual acuity value VAbn.

By designing the progressive addition lens after performing both the method for changing the design type of the distance portion and the method for changing the design type of the near portion, the progressive addition lenses which are more suitable for the expected wearer can be provided.

The exemplary embodiment of the present invention has been described above. The embodiment of the present invention is not limited to the one described above, and various modification can be performed without departing the scope of the technical idea of the present invention. For example, embodiments and modifications described in the specification as examples or a content in which the obvious embodiments and modifications are appropriately combined are included in the embodiment of the present application.

In the embodiment, the blur sensitivity of the expected wearer is estimated based on the relation (difference) between the full corrected value determined by the optometric work and the value of the prescription power adjusted and determined by the optometric work. However, in another embodiment, the blur sensitivity may be measured by using a near-infrared spectroscopy (NIRS) brain measuring device.

For example, details of the measuring method for the blur sensitivity by using the NIRS brain measuring device is disclosed in WO 2014/22926 A (referred to as "International Patent Publication 1"). As an example, when measuring the blur sensitivity by using the NIRS brain measuring device, the expected wearer wears the trial lens having the prescription power (distance power) determined by the optometric work. Also, a probe of the NIRS brain measuring device is set to a forehead of the expected wearer (for example, refer to Fig. 3 in International Patent Publication 1). A plurality of images having different sharpness (image clarity level) is randomly presented to the expected wearer having the probe set thereto. The power of the trial lens is appropriately adjusted according to the distance between the expected wearer and the presented image. The presented images include an original image of which the sharpness is not adjusted and a sharpened image and blurred image to the original image. A specific example of the presented images is referred in Fig. 22 and Fig. 25 in International Patent Publication 1.

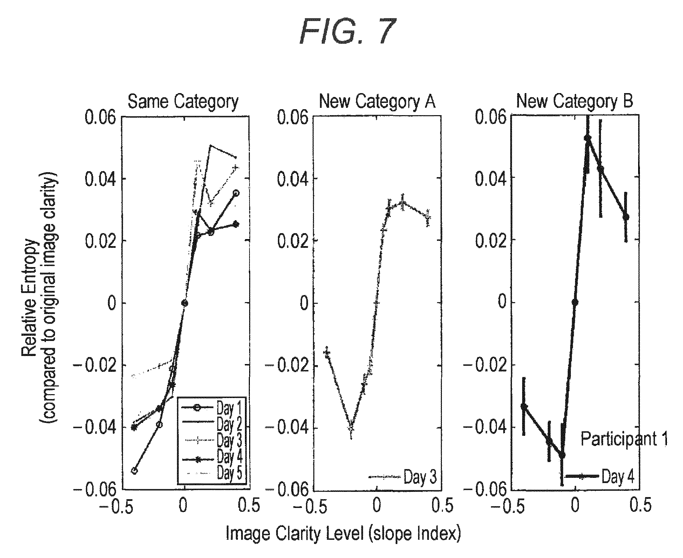

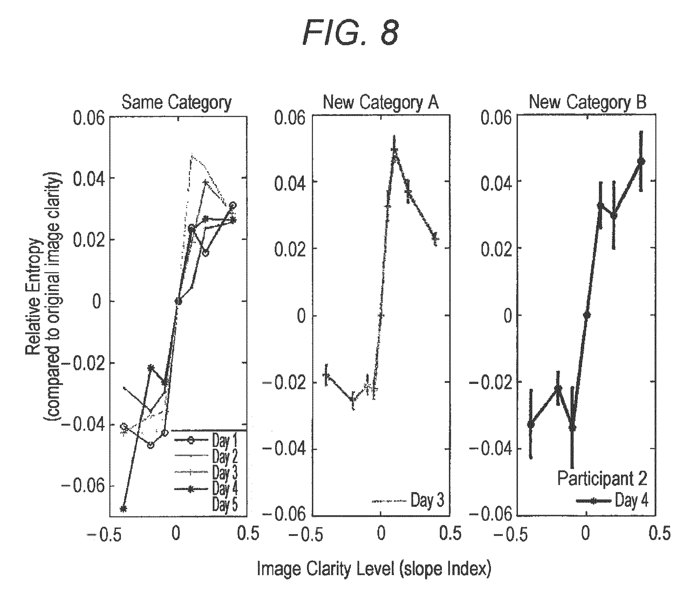

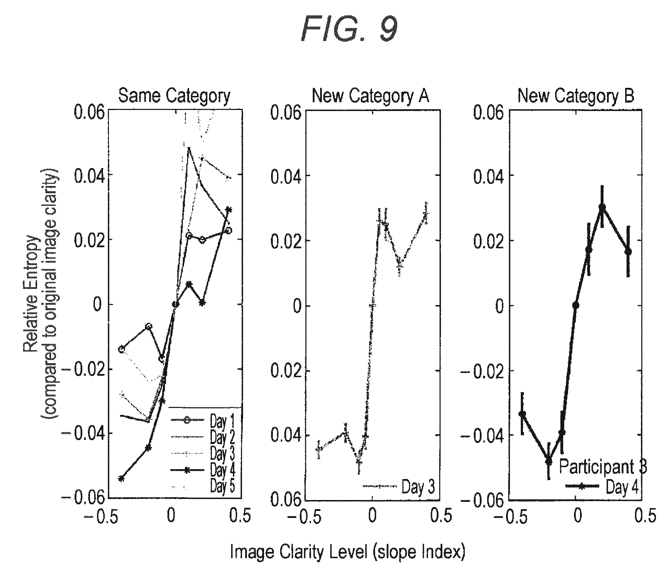

Figs. 23A to 23C in International Patent Publication 1 are cited as Figs. 7 to 9. Each of Figs. 7 to 9 is a graph for exemplifying the entropy for each subject (expected wearer) according to the sharpness of the presented image. In Figs. 7 to 9, the vertical axis indicates a relative entropy, and the value of the entropy of the expected wearer when looking at the original image is assumed to be the reference (entropy is zero). The horizontal axis indicates the sharpness (image clarity level (slope index)) of the presented image. In Figs. 7 to 9, the sharpness of the original image is zero, and the sharpness of the sharpened image relative to the original image is larger than zero (positive value), and the sharpness of the blurred image relative to the original image is smaller than zero (negative value). In Figs. 7 to 9, the relative entropy is determined based on a biological signal (intracerebral blood (amount of oxygenated hemoglobin)) measured by the NIRS brain measuring device. In the measuring method for the blur sensitivity by using the NIRS brain measuring device in International Patent Publication 1, when the inclination of the graph in each of Figs. 7 to 9 is larger (when reaction of brain of expected wearer at the time when image is slightly sharpened or slightly blurred is larger), the evaluation is made such that the blur sensitivity is high. That is, by employing this measuring method, the blur sensitivity of the expected wearer is objectively evaluated based on the intracerebral blood.

Here, an exemplary determination criterion to determine the level of the blur sensitivity is described. For example, the determination criterion is determined based on the measurement results of about 20 subjects. Specifically, the relative entropy at the time when the slope index is changed by ± 0.1 having the original image (slope index=0) as a reference and the subject looks at the image sharpened or blurred by image processing is measured. Next, an average change condition of the relative entropy (inclination in graph in Figs. 7 to 9) is obtained based on the measurement results of the subjects. For convenience of description, the average change condition of the relative entropy is referred to as a "reference change condition" below. When the relative entropy of the expected wearer is measured and the change condition is obtained, the change condition is compared with the reference change condition. When the change condition of the relative entropy of the expected wearer is larger than the reference change condition (for example, decrease in the relative entropy of the expected wearer is larger than that in the average relative entropy), it is determined that the blur sensitivity is high. When the change condition is smaller than the reference change condition (for example, decrease in the relative entropy of the expected wearer is smaller than that in the average relative entropy), it is determined that the blur sensitivity is low.

It is preferable to measure larger number of subjects to obtain the determination criteria (reference change condition). However, when about 20 subjects are measured, an appropriate and reasonable reference change condition is obtained. However, it is necessary for each subject to have a sufficient vision relative to the presented image. For having a sufficient vision, it is necessary to measure the vision of the subject after the vision is appropriately corrected by using the spectacle lenses and the like. Also, when the reference change condition is obtained, the degree and range of the change of the relative entropy are changed according to the size of the presented image for the subject and the presented image. Therefore, it is preferable that the presented image at the time when the reference change condition is obtained be the same as that at the time of performing the measurement of the expected wearer.

Also, based on the level of the reaction relative to the image (slope index > 0) more sharpened than the original image (slope index = 0) (change condition of relative entropy of expected wearer) and the level of the reaction relative to the image (slope index<0) more blurred than the original image (slope index=0) (change condition of relative entropy of expected wearer), the following three points can be evaluated. The three points include (1) whether the prescription power approximates to the full corrected value, (2) whether the prescription power is adjusted to the positive power side than the full corrected value, and (3) whether the blur sensitivity of the expected wearer is high or low. In this evaluation, an image in which the slope index is changed by $\pm$ 0.05 such as the original image (slope index = 0) is presented to the expected wearer. The evaluation is performed in a range in which the image is slightly sharpened or blurred.

When the increase in the entropy relative to the sharpened image to the original image is smaller (change condition of relative entropy of expected wearer is smaller), it is considered that the expected wearer wears the lenses with the prescribed power and can sufficiently perceive the sharpened image. Also, when the decrease in the entropy relative to the blurred image to the original image is smaller (change condition of relative entropy of expected wearer is smaller), it is considered that the expected wearer wears the lenses with the prescribed power and can perceive the blurred image as the image which is not blurred. In this case, it is evaluated that the prescription power approximates to the full corrected value (1). In an opposite way, when the increase in the entropy relative to the sharpened image to the original image is larger (change condition of relative entropy of expected wearer is larger), it is considered that the expected wearer wears the lenses with the prescribed power and requires the more sharpened image. Also, when the decrease in the entropy relative to the blurred image to the original image is larger (change condition of relative entropy of expected wearer is larger), it is considered that the expected wearer wears the lenses with the prescribed power and perceives the more blurred image as a largely blurred image. In this case, it is evaluated that the prescription power is adjusted to the positive power side than the full corrected value (2). Also, regarding (3), when the reaction relative to the blurred image to the original image is small, it is evaluated that the blur sensitivity of the expected wearer is low. When the reaction relative to the blurred image to the original image is large, it is evaluated that the blur sensitivity of the expected wearer is high.

Next, for example, a case is considered where the visual acuity level of the expected wearer through the distance portion is high and it is evaluated that the blur sensitivity of the expected wearer relative to the presented image at a far distance is low. In this case, similarly to case 1 in the embodiment, the range of the basic design lens to be selected is shifted from the range SDL to the range SDL' on the distance soft side as illustrated in Fig. 6A.

Whether the visual acuity level of the expected wearer is high or not by using the prescription power may be determined, for example, based on the integral of the entropy's power spectrum density (IPSD) (refer to Figs. 27 to 32 in International Patent Publication 1).

Also, for example, a case is considered where the visual acuity level of the expected wearer through the distance portion is high and it is evaluated that the blur sensitivity of the expected wearer relative to the presented image at the far distance is high. In this case, similarly to case 2 in the embodiment, the range of the basic design lens to be selected is shifted from the range SDL to the range SDL' on the distance clear side as illustrated in Fig. 6B.

Also, for example, a case is considered where the visual acuity level of the expected wearer through the distance portion is not high and it is evaluated that the blur sensitivity of the expected wearer relative to the presented image at the far distance is low. In this case, similarly to case 3 in the embodiment, the range of the basic design lens to be selected is not shifted from the range SDL as illustrated in Fig. 6C.

Also, for example, a case is considered where the visual acuity level of the expected wearer through the distance portion is not high and it is evaluated that the blur sensitivity of the expected wearer relative to the presented image at the far distance is high. In this case, similarly to case 4 in the embodiment, the range of the basic design lens to be selected is shifted from the range SDL to the range SDL' on the distance clear side as illustrated in Fig. 6D.

**Claims**

1. A device for manufacturing a progressive lens, comprising:

    a blur sensitivity estimating unit configured to estimate a blur sensitivity indicating a sensitivity of a spectacles wearer relative to a blur at the time when the spectacles wearer looks at an object through lenses having a prescription power of a first refractive unit;
    a first area adjusting unit configured to adjust an area of a clear-visible region of the first refractive unit based on the blur sensitivity estimated by the blur sensitivity estimating unit;
    a design reference lens selecting unit configured to select at least a single basic design lens for coinciding with or approximating to the lens of which the area of the clear-visible region of the first refractive unit is adjusted by the first area adjusting unit from among a predetermined basic design lens group as a design reference lens of the progressive lens to be worn by the spectacles wearer; and

a data creating unit configured to create data to manufacture the progressive lens based on a lens design of the design reference lens selected by the design reference lens selecting unit, **characterized in that** the progressive lens includes the first refractive unit having a first refractive power, a second refractive unit having a second refractive power in which a refractive power (addition refractive power) is applied to first refractive power for near vision, and a corridor in which the refractive power is progressively changed along a meridian from the first refractive unit to the second refractive unit.

2. The device for manufacturing a progressive lens according to claim 1, **characterized in that** the first area adjusting unit adjusts the area of the clear range of the first refractive unit based on the blur sensitivity based on the blur sensitivity estimating unit and a visual acuity obtained by using the prescription power of the first refractive unit.

3. The device for manufacturing a progressive lens according to claim 1 or 2, comprising a second area adjusting unit configured to adjust an area of a clear-visible region of the second refractive unit based on a visual acuity obtained by using the prescription power of the second refractive unit, **characterized in that** the design reference lens selecting unit selects at least a single basic design lens for coinciding with or approximating to the lens of which the area of the clear range of the first refractive unit is adjusted by the first area adjusting unit and for coinciding with or approximating to the lens of which the area of the clear-visible region of the second refractive unit adjusted by the second area adjusting unit from among the basic design lens group as a design reference lens of the progressive lens to be worn by the spectacles wearer.

4. The device for manufacturing a progressive lens according to any one of claims 1 to 3, **characterized in that** the blur sensitivity is estimated based on a relation between a full corrected value of the spectacles wearer and the value of the prescription power of the first refractive unit.

5. The device for manufacturing a progressive lens according to claim 4, comprising:

an input unit configured to request inputs of the full corrected value and the value of the prescription power of the first refractive unit, **characterized in that** the blur sensitivity estimating unit estimates the blur sensitivity based on a relation between the full corrected value input via the input unit and the value of the prescription power of the first refractive unit input via the input unit.

6. The device for manufacturing a progressive lens according to claim 5 referring to claim 2, **characterized in that** the input unit requests an input of the visual acuity obtained by using the prescription power of the first refractive unit, and

the first area adjusting unit adjusts the area of the clear-visible region of the first refractive unit based on the blur sensitivity estimated by the blur sensitivity estimating unit and the visual acuity obtained by using the prescription power of the first refractive unit input via the input unit.

7. A lens supply system for supplying a progressive lens, comprising:

a device configured to manufacture a progressive lens according to any one of claims 1 to 4; and an input device configured to be connected to the device via a predetermined network, **characterized in that** the input device includes an input unit for requesting an input of information to design the progressive lens and a transmission unit for transmitting the information input via the input unit to the device, the blur sensitivity estimating unit estimates the blur sensitivity based on the information transmitted by the transmission unit, the first area adjusting unit adjusts the area of the first refractive unit based on the blur sensitivity estimated by the blur sensitivity estimating unit, and the design reference lens selecting unit selects at least a single basic design lens for coinciding with or approximating to the lens of which the area of the first refractive unit is adjusted by the first area adjusting unit from among a predetermined basic design lens group as a design reference lens of the progressive lens to be worn by a spectacles wearer.

8. The lens supply system for supplying a progressive lens according to claim 7, **characterized in that**

the input unit requests inputs of the full corrected value and the value of the prescription power of the first refractive unit, and

the blur sensitivity estimating unit estimates the blur sensitivity based on a relation between the full corrected value transmitted by the transmission unit and the value of the prescription power of the first refractive unit transmitted by the transmission unit.

**9.** The lens supply system for supplying a progressive lens according to claim 8, **characterized in that**
the input unit requests the input of the visual acuity obtained by using the prescription power of the first refractive unit, and

the first area adjusting unit adjusts the area of the clear range of the first refractive unit based on the blur sensitivity estimated by the blur sensitivity estimating unit and the visual acuity obtained by using the prescription power of the first refractive unit transmitted by the transmission unit.

**10.** A method for manufacturing a progressive lens, comprising:

a blur sensitivity estimating step of estimating a blur sensitivity indicating a sensitivity of a spectacles wearer relative to a blur at the time when the spectacles wearer looks at an object through lenses having a prescription power of a first refractive unit;
a first area adjusting step of adjusting an area of the first refractive unit based on the blur sensitivity estimated in the blur sensitivity estimating step;
a design reference lens selecting step of selecting at least a single basic design lens for coinciding with or approximating to the lens of which the area of the first refractive unit is adjusted in the first area adjusting step from among a predetermined basic design lens group as a design reference lens of the progressive lens to be worn by the spectacles wearer; and
a data creating step of creating data to manufacture the progressive lens based on a lens design of the design reference lens selected in the design reference lens selecting step, **characterized in that**
the progressive lens includes the first refractive unit having a first refractive power, a second refractive unit having a second refractive power in which a refractive power (addition refractive power) is applied to the first refractive power for near vision, and a corridor in which the refractive power is progressively changed along a meridian from the first refractive unit to the second refractive unit.

**11.** The method for manufacturing a progressive lens according to claim 10, **characterized in that**
in the first area adjusting step, the area of the clear-visible region of the first refractive unit is adjusted based on the blur sensitivity estimated in the blur sensitivity estimating step and a visual acuity obtained by using the prescription power of the first refractive unit.

**12.** The method for manufacturing a progressive lens according to claim 10 or 11, comprising:

a second area adjusting step of adjusting an area of the second refractive unit based on the visual acuity obtained by using the prescription power of the second refractive unit, **characterized in that**
in the design reference lens selecting step, at least a single basic design lens for coinciding with or approximating to the lens of which the area of the first refractive unit is adjusted in the first area adjusting step and for coinciding with or approximating to the lens of which the area of the second refractive unit is adjusted in the second area adjusting step from among the basic design lens group as a design reference lens of the progressive lens to be worn by the spectacles wearer.

**13.** The method for manufacturing a progressive lens according to any one of claims 10 to 12, **characterized in that**
the blur sensitivity is estimated based on a relation between a full corrected value of the spectacles wearer and the value of the prescription power of the first refractive unit.

**14.** A manufacturing method for a progressive lens, comprising:

a step of manufacturing a progressive lens by using data created by performing the method according to any one of claims 10 to 13.

**15.** A program causing a computer to perform the method according to any one of claims 10 to 13.

**16.** A lens supply system for supplying a progressive lens, comprising:

a device configured to include a blur sensitivity estimating unit which estimates a blur sensitivity indicating a sensitivity of a spectacles wearer relative to a blur at the time when the spectacles wearer looks at an object through lenses having a prescription power of a first refractive unit, a first area adjusting unit which adjusts an area of a clear-visible region of the first refractive unit based on the blur sensitivity estimated by the blur sensitivity estimating unit, and a design reference lens selecting unit which selects at least a single basic design lens for coinciding with or approximating to the lens of which the area of the clear-visible region of the first refractive unit is adjusted by the first area adjusting unit from among a predetermined basic design lens group as a design reference lens of the progressive lens to be worn by the spectacles wearer, and a data creating unit which creates data to manufacture the progressive lens based on a lens design of the design reference lens selected by the design reference lens selecting unit; and

a biological signal measuring device configured to measure a biological signal of the spectacles wearer according to sharpness of a presented image, **characterized in that**

the blur sensitivity is estimated based on the biological signal measured by the biological signal measuring device, and

the progressive lens includes the first refractive unit having a first refractive power, a second refractive unit having a second refractive power in which a refractive power (addition refractive power) is applied to first refractive power for near vision, and a corridor in which the refractive power is progressively changed along a meridian from the first refractive unit to the second refractive unit.

17. The lens supply system for supplying a progressive lens according to claim 16, **characterized in that**
the first area adjusting unit adjusts the area of the clear range of the first refractive unit based on the blur sensitivity estimated by the blur sensitivity estimating unit and a vision obtained by using the prescription power of the first refractive unit.

18. The lens supply system for supplying a progressive lens according to claim 16 or 17, comprising
a second area adjusting unit configured to adjust an area of a clear-visible region of the second refractive unit based on a vision obtained by using the prescription power of the second refractive unit, **characterized in that**
the design reference lens selecting unit selects at least a single basic design lens for coinciding with or approximating to the lens of which the area of the clear range of the first refractive unit is adjusted by the first area adjusting unit and for coinciding with or approximating to the lens of which the area of the clear-visible region of the second refractive unit is adjusted by the second area adjusting unit from among the basic design lens group as a design reference lens of the progressive lens to be worn by the spectacles wearer.

19. A method for manufacturing a progressive lens, comprising:

a biological signal measuring step of measuring a biological signal of a spectacles wearer according to sharpness of a presented image;
a blur sensitivity estimating step of estimating a blur sensitivity indicating a sensitivity of the spectacles wearer relative to a blur at the time when the spectacles wearer looks at an object through lenses having a prescription power of a first refractive unit based on the biological signal measured in the biological signal measuring step;
a first area adjusting step of adjusting an area of the first refractive unit based on the blur sensitivity estimated in the blur sensitivity estimating step;
a design reference lens selecting step of selecting at least a single basic design lens for coinciding with or approximating to the lens of which the area of the first refractive unit is adjusted in the first area adjusting step from among a predetermined basic design lens group as a design reference lens of the progressive lens to be worn by the spectacles wearer; and
a data creating step of creating data to manufacture the progressive lens based on a lens design of the design reference lens selected in the design reference lens selecting step, **characterized in that**
the progressive lens includes the first refractive unit having a first refractive power, a second refractive unit having a second refractive power in which a refractive power (addition refractive power) is applied to the first refractive power for near vision, and a corridor in which the refractive power is progressively changed along a meridian from the first refractive unit to the second refractive unit.

20. The method for manufacturing a progressive lens according to claim 19, **characterized in that**
in the first area adjusting step, the area of the clear-visible region of the first refractive unit is adjusted based on the blur sensitivity estimated in the blur sensitivity estimating step and a visual acuity obtained by using the prescription power of the first refractive unit.

**21.** The method for manufacturing a progressive lens according to claim 19 or 20, comprising:

a second area adjusting step of adjusting an area of the second refractive unit based on the visual acuity obtained by using the prescription power of the second refractive unit, **characterized in that**
in the design reference lens selecting step, at least a single basic design lens for coinciding with or approximating to the lens of which the area of the first refractive unit is adjusted in the first area adjusting step and for coinciding with or approximating to the lens of which the area of the second refractive unit is adjusted in the second area adjusting step is selected from among the basic design lens group as a design reference lens of the progressive lens to be worn by the spectacles wearer.

**22.** A manufacturing method for a progressive lens, comprising:

a step of manufacturing a progressive lens by using data created by performing the method according to any one of claims 19 to 21.

**23.** A program causing a computer to perform the method according to any one of claims 19 to 21.

# FIG. 1

10

1

SPECTACLES STORE

SHOP COMPUTER ──100

SPECTACLE SELECTOR APPLICATION ──102

20

SPECTACLE LENS MANUFACTURING PLANT

HOST COMPUTER ~200

SPECTACLE LENS DESIGNING COMPUTER

SPECTACLE LENS PROCESSING COMPUTER ~204

202

PROCESSING MACHINE

208

LENS CASTING DEVICE

206

# FIG. 2

EP 3 088 938 A1

(a)

## STEP1 PRESCRIPTION POWER INFORMATION

### PRESCRIPTION POWER

|   | Sph | Cyl | Ax | Add |
|---|-----|-----|-----|-----|
| R | −2.25 | −0.75 | 90 | 2.25 |
| L | −2.75 | −1.25 | 110 | 2.25 |

### FULL CORRECTED VALUE

|   | Sph | Cyl | Ax | Add |
|---|-----|-----|-----|-----|
| R | −2.75 | −1.25 | 90 | 2.25 |
| L | −3.25 | −1.25 | 110 | 2.25 |

### VISION OBTAINED BY USING PRESCRIPTION POWER

|   | DISTANT POINT | NEAR POINT |
|---|---------------|------------|
| R | 1.0 | 0.6 |
| L | 1.2 | 0.5 |

### VISION OBTAINED BY USING FULL CORRECTED VALUE

|   | DISTANT POINT |
|---|---------------|
| R | 1.2 |
| L | 1.5 |

(b)

## STEP3 INPUT VALUES

### INPUT VALUES OF LENS SCALE APP

| | |
|---|---|
| FRAME (VERTEX DISTANCE) | 8.2mm |
| PANTOSCOPIC ANGLE | 6.2° |
| FRAME FACE FORM ANGLE | 3.9° |
| LENS VERTICAL WIDTH | 32.2mm |
| FITTING POINT | 4mm |
| LENS LAYOUT "CD" R | 27.8mm |
| LENS LAYOUT "CD" L | 26.4mm |

(c)

## STEP4 BASIC LIFE STYLE

* PLEASE ANSWER ALL QUESTIONS

| | VERY IMPORTANT | IMPORTANT | ORDINARY | NOT VERY IMPORTANT | NOT IMPORTANT |
|---|---|---|---|---|---|
| ● IMPORTANCE LEVEL TO SEE DISTANT POINT IN YOUR LIFE | ☐ | ◉ | ☐ | ☐ | ☐ |
| ● IMPORTANCE LEVEL TO SEE NEAR POINT IN YOUR LIFE | ☐ | ☐ | ◉ | ☐ | ☐ |

● TYPE OF COMPUTER THAT YOU USE AND IMPORTANCE LEVEL

| | VERY IMPORTANT | IMPORTANT | ORDINARY | NOT VERY IMPORTANT | NOT IMPORTANT |
|---|---|---|---|---|---|
| NOTE BOOK TYPE | ☐ | ◉ | ☐ | ☐ | ☐ |
| DESK TOP TYPE | ☐ | ☐ | ◉ | ☐ | ☐ |
| TABLET TYPE AND SMARTPHONE | ☐ | ☐ | ☐ | ☐ | ◉ |
| ● IMPORTANCE LEVEL TO DRIVE CAR AND BIKE IN YOUR LIFE | ☐ | ☐ | ☐ | ◉ | ☐ |

(d)

## RESULTS RESULTS

DATE MAY, 30 2013

### RECOMMENDED PROGRESSIVE LENS FOR YOU

| TYPE | CORRIDOR LENGTH |
|------|-----------------|
| B−3 | 14mm |

## FIG. 3

```
( MANUFACTURING PROCESS )
            │
            ▼
┌──────────────────────────────────────┐  S11
│      DESIGNING SPECTACLE LENS         │
└──────────────────────────────────────┘
            │
            ▼
┌──────────────────────────────────────┐  S12
│    MANUFACTURING SPECTACLE LENS       │
└──────────────────────────────────────┘
            │
            ▼
        (   END   )
```

## FIG. 4

```
            (  START  )
                │
                ▼
┌──────────────────────────────────────────────────┐  S21
│ CALCULATE EACH BASIC DESIGN POINT DES1 TO DES7     │
└──────────────────────────────────────────────────┘
                │
                ▼
┌──────────────────────────────────────────────────┐  S22
│   CALCULATE EIGHTH BASIC DESIGN POINT DES8          │
└──────────────────────────────────────────────────┘
                │
                ▼
┌──────────────────────────────────────────────────┐  S23
│   CALCULATE BASIC DESIGN POINT DES BY USING         │
│   CALCULATED BASIC DESIGN POINTS DES1 TO DES8       │
└──────────────────────────────────────────────────┘
                │
                ▼
┌──────────────────────────────────────────────────┐  S24
│ SELECT BASIC DESIGN LENS FROM AMONG PREDETERMINED   │
│ BASIC DESIGN LENS GROUP BASED ON CALCULATED         │
│ BASIC DESIGN POINT DES AND DISPLAY SELECTED         │
│ BASIC DESIGN LENS ON SCREEN                         │
└──────────────────────────────────────────────────┘
                │
                ▼
            (   END   )
```

FIG. 5

FIG. 6

## FIG. 7

## FIG. 8

EP 3 088 938 A1

# FIG. 9

## INTERNATIONAL SEARCH REPORT

| International application No. |
| --- |
| PCT/JP2014/084550 |

A. CLASSIFICATION OF SUBJECT MATTER
*G02C13/00*(2006.01)i, *G02C7/06*(2006.01)i

According to International Patent Classification (IPC) or to both national classification and IPC

B. FIELDS SEARCHED

Minimum documentation searched (classification system followed by classification symbols)
G02C13/00, G02C7/06

Documentation searched other than minimum documentation to the extent that such documents are included in the fields searched
Jitsuyo Shinan Koho          1922-1996   Jitsuyo Shinan Toroku Koho   1996-2015
Kokai Jitsuyo Shinan Koho    1971-2015   Toroku Jitsuyo Shinan Koho   1994-2015

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X<br>Y<br>A | JP 2012-523590 A  (Essilor International<br>(Compagnie Generale d'Optique)),<br>04 October 2012 (04.10.2012),<br>entire text; all drawings<br>& US 2012/0038889 A1    & EP 2419782 A<br>& WO 2010/119218 A1    & FR 2944364 A<br>& CA 2758690 A | 1-3,7,10-12,<br>14-15<br>16-23<br>4-6,8-9,13 |
| Y<br>A | WO 2010/035726 A1  (Tokai Optical Co., Ltd.),<br>01 April 2010 (01.04.2010),<br>paragraph [0007]<br>& JP 5205580 B        & US 2011/0170058 A1<br>& EP 2348350 A1       & CN 102165362 A<br>& KR 10-2011-0067102 A  & TW 201013248 A | 16-23<br>1-15 |

| ☒ | Further documents are listed in the continuation of Box C. | ☐ | See patent family annex. |
| --- | --- | --- | --- |

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered    to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>    11 March 2015 (11.03.15) | Date of mailing of the international search report<br>    24 March 2015 (24.03.15) |
| --- | --- |
| Name and mailing address of the ISA/<br>    Japan Patent Office<br>    3-4-3,Kasumigaseki,Chiyoda-ku,<br>    Tokyo 100-8915,Japan | Authorized officer<br><br>Telephone No. |

Form PCT/ISA/210 (second sheet) (July 2009)

**INTERNATIONAL SEARCH REPORT**

International application No.

PCT/JP2014/084550

C (Continuation).    DOCUMENTS CONSIDERED TO BE RELEVANT

| Category* | Citation of document, with indication, where appropriate, of the relevant passages | Relevant to claim No. |
|---|---|---|
| A | JP 2012-233933 A  (Tokai Optical Co., Ltd.), 29 November 2012 (29.11.2012), entire text; all drawings (Family: none) | 1-23 |

Form PCT/ISA/210 (continuation of second sheet) (July 2009)

**EP 3 088 938 A1**